# EUROPEAN PATENT APPLICATION

(11) **EP 0 901 798 A1**
(43) Date of publication of application: **17.03.1999**
(21) Application number: 98111658.5
(22) Date of filing: 24.06.1998
(51) Int. Cl.: A61M 16/00, G06F 3/03

(54) **Breathing apparatus**

(30) Priority: 21.08.1997 SE 9703016
(71) Applicant: Siemens-Elema AB, 171 95 Solna (SE)
(72) Inventor: Klingberg, Wilhelm, 161 55 Bromma (SE)

(57) **Abstract**

A breathing apparatus, comprising a user interface (8) for programming the breathing apparatus's operating mode, said user interface (8) comprising an interactive screen (10) with a touch-sensitive surface capable of displaying at least active programming and indicated programming, devised so at least some programming can be performed when specially arranged segments on the screen (10) are touched one or more times, and a verification switch (12) for implementing active storage in the breathing apparatus (2) of indicated programming displayed on the screen (10). To prevent failure to verify indicated programming, the user interface (8) is devised so the need to verify indicated programming is brought to the attention of the operator. This can be performed, according to the invention, when indicated programming, e.g. a change in some parameter, is displayed on the screen (10) with an appearance that differs from the appearance of the normal presentation pattern (18) for active programming (24B, 24B) until the verification switch (12) is actuated to implement active storage of the indicated programming. Or an error message or a message requesting verification by the operator can be activated for display on the screen.

## Description

The present invention relates to a breathing apparatus according to the preambles to claims 1, 5, 7 and 10.

Swedish patent application No. SE 9600090 describes a breathing apparatus with an input unit and an output unit for entering instructions (programming) for a control unit in the breathing apparatus. The input unit forms a user interface which can display the active programming, i.e. the operating mode (such as Pressure Control, Volume Control, etc.) selected by the operator and the parameters (such as pressure, flow, inspiratory time, expiratory time, ratio of inspiratory time and expiratory time, etc.) programmed by the operator. The user interface encompasses an interactive, touch-sensitive screen and a verification switch. The operator uses them for changing active programming. Altered parameters are displayed on the screen and constitute what this application refers to as 'indicated programming', i.e. an intended change which has not yet been sent to the control unit by verification with the verification switch.

Since indicated programming is displayed, instead of active programming, the operator can perform certain operations without verifying any changes made, so entered changes do not become active programming. In the worst instance, a patient connected to the breathing apparatus might be subjected to treatment which does not fully agree with the operator's intentions.

One objective of the invention is to achieve a breathing apparatus according to the above which eliminates the described problems.

The objective is achieved in accordance with the invention in that the apparatus is devised according to the characterising part to claim 1.

The objective is also achieved in accordance with the invention in that the breathing apparatus is devised according to the characterising part to claim 5.

The objective is also achieved in accordance with the invention in that the breathing apparatus is devised according to the characterising part to claim 7.

The objective is also achieved in accordance with the invention in that the breathing apparatus is devised according to the characterising part to claim 10.

Advantageous embodiments will be evident from the respective dependent claims to the independent claims.

Embodiments of the breathing apparatus according to the invention will be described below in greater detail, referring to attached drawings in which
FIG. 1 shows a breathing apparatus according to the invention;
FIG. 2 shows a first embodiment of a user interface in the breathing apparatus;
FIG. 3 shows a second embodiment of a user interface in the breathing apparatus, and
FIG. 4 shows a third embodiment of a user interface in the breathing apparatus.

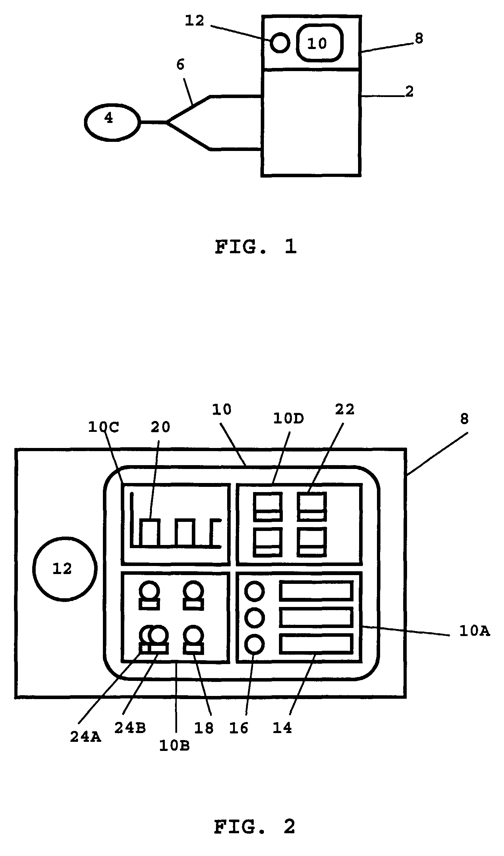

The breathing apparatus 2 in FIG. 1 supplies a patient 4 with a breathing gas via a tubing system 6. In principle, the breathing apparatus 2 can consist of a ventilator/ respirator/anaesthesia machine. These devices are well-known, and no description of their function and modes of operation is necessary here.

The breathing apparatus 2 includes a user interface 8 for programming the breathing apparatus 2 with the desired mode of operation (e.g. Pressure Control (PC), Pressure Support (PS) or Volume Control (VC), etc.) and values for parameters (e.g. oxygen concentration, pressure, flow, inspiratory time, expiratory time, etc.) . The user interface 8 can be a completely integrated component of the breathing apparatus 2 or be attached to it as an external module.

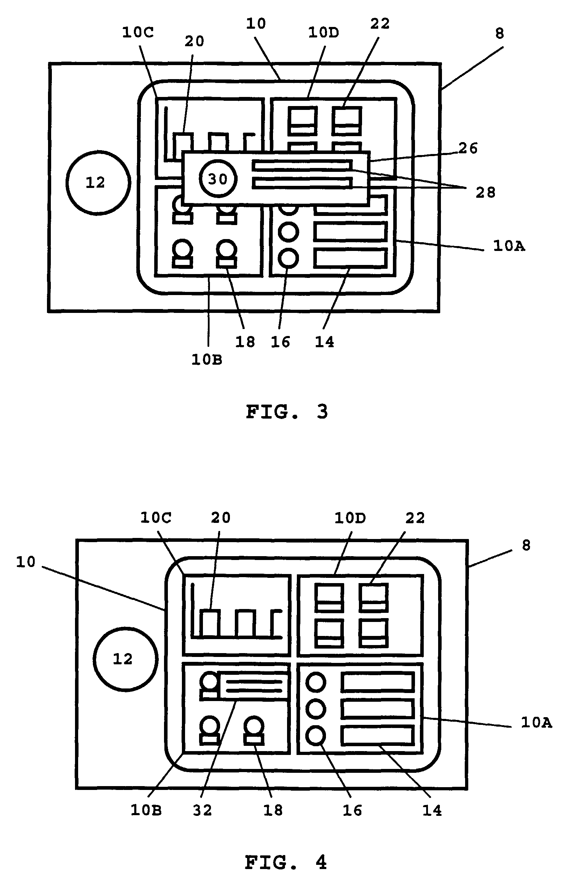

The user interface 8 comprises an interactive screen 10 and a setting knob 12. As shown in FIG. 2, the screen 10 can have a graphical interface making it easier for the operator to select a program and check the operation of the breathing apparatus. In this instance, the screen 10 has been divided into four graphical fields 10A, 10B, 10C, 10D. The first graphical field 10A holds text 14 indicating the operating mode that can be selected by touching the corresponding activation area 16. The second graphical field 10B contains the parameters that can be set by touching the respective parameter designation 18. The value can be set in plurality of ways. The setting knob 12 can e.g. be devised to change the displayed value, when the knob is rotated. The altered value is stored in the breathing apparatus's control unit when the setting knob 12 is pressed (the knob then functioning as a verification switch). The third graphical field 10C holds a graphical diagram 20 of one or a plurality of chronological sequences in the breathing apparatus, e.g. pressure and flow sequences. The fourth graphical field 10D displays various measurement values, e.g. oxygen concentration, carbon dioxide level etc. in different display fields 22. The screen 10 can also be devised to individually display one or more of the graphical fields 10A, 10B, 10C, 10D.

When an operating mode and the associated parameters are programmed, the user interface 8 can be devised to suggest the parameters on the basis of information stored on the patient. Default parameters can also be stored in the user interface 8. Programming can even be performed during ongoing treatment.

Irrespective of the mode employed, active programming is generally in force when the operator is to tailor the program to the patient. The mode prevailing from the time the operator enters a change shown on the screen 10 until the new programming is acknowledged (thereby transforming it into active programming) is referred to as 'indicated programming'. To avoid problems, the difference between 'active' programming and 'indicated' programming must be brought to the operator's attention so that the indicated programming is inadvertently not acknowledged (verified) with the verification switch 12.

The example of the first embodiment of the user interface 8 in FIG. 2 shows how this could be accomplished. Assume that the value of an active parameter 24 in the second graphical field 10B is to be changed. Since both the active parameter 24A and the indicated parameter 24B are displayed, the operator will realise that the change has not been acknowledged or verified. The active parameter 24A and the indicated parameter 24B can be displayed with different graphical features, e.g. in different colours or with one blinking and the other steady.

When a different colour or blinking is employed in the graphical interface, displaying only the indicated parameter 24B may suffice.

FIG. 3 shows a second embodiment of the user interface 8. All components and graphical interfaces identical to those in the first embodiment have been assigned the same designation numerals. So a repetition of what the different graphical fields 10A, 10B, 10C, 10D display is not necessary. In this instance, a graphical error message 26 containing text information 28 is utilised. The error message can be activated and displayed on the screen 10, e.g. when a specific time elapses after a parameter is changed without verification with the verification switch 12. As an alternative, or in combination therewith, the error message can be generated if the operator attempts to switch to another graphical interface or some other programming without first verifying the indicated parameter.

Here, the interactive screen 10 can be used so verification is performed when a verification area 30 in the error message 26 is touched. Alternately, the error message can be devised in such a way that verification must be performed with the verification switch 12. If the indicated program is not to be used (not made active), the field 30 must be touched for a return to active programming.

It would also be possible (in all disclosed embodiments) to integrate the switch 12 physically with the interactive screen 10 or to create a touch sensible image of the switch 12 on the interactive screen 10. A touch sensible image of the switch 12 could, for instance, be operated by having increments on one side of the image, decrements on the other side and verification in the middle. The image need not be in the shape of a knob.

A third embodiment of the user interface 8 is shown in FIG. 4. Once again, all components and graphical interfaces which are identical to those in previous embodiments have the same designation numerals. A graphical verification request 32 is utilised in the third embodiment. This request is activated and displayed on the screen 10 as soon as an operator enters indicated programming. The graphical verification request 32 remains on display until verification occurs.

Different embodiments can be combined. For example, an error message can also be displayed in the first and third embodiments after a specific time elapses without any verification.

There are naturally numerous ways in which the interface can be devised, even for breathing apparatuses with a limited number of operating modes and parameters. The aim of the invention is in particular to ensure that the operator of the breathing apparatus is made aware of the need to verify or acknowledge any indicated programming entered in order to activate same. This can be done for a large number of graphical interfaces (not shown) without departing from the main principles of the invention.

## Claims

1. A breathing apparatus (2), comprising a user interface (8) for programming the breathing apparatus's (2) operating mode, said user interface (8) comprising an interactive screen (10) with a touch-sensitive surface capable of displaying at least active programming and indicated programming, devised so at least some programming can be performed when certain segments of the screen (10) are touched one or more times, and a verification switch (12) for implementing active storage in the breathing apparatus (2) of indicated programming displayed on the screen (10), **characterised in** that the user interface (8) is designed so indicated programming, e.g. a change in some parameter, is displayed on the screen (10), with an appearance that differs from the appearance of the normal presentation pattern (18) for active programming (24B, 24B), until the verification switch (12) is actuated to implement active storage of the indicated programming.

2. A breathing apparatus according to claim 1, **characterised in** that the screen simultaneously displays indicated programming (24B) and active programming (24A).

3. A breathing apparatus according to claims 1 or 2, **characterised in** that the screen displays indicated programming (24B) and/or active programming (24A) in a different colour(s).

4. A breathing apparatus according to any of the above claims, **characterised in** that the screen displays indicated programming (24B) and/or active programming (24A) with blinking characters.

5. A breathing apparatus (2), comprising a user interface (8) for programming the breathing apparatus's (2) operating mode, said user interface (8) comprising an interactive screen (10) with a touch-sensitive surface, capable of displaying at least active programming and indicated programming, devised so at least some programming can be performed when specially arranged segments on the screen (10) are touched one or more times, and a verification switch (12) for implementing active storage in the breathing apparatus (2) of the indicated programming displayed on the screen (10), **characterised in** that the user interface (8) is devised to display an error message (26) on the screen if the verification switch (12) is not actuated within a predetermined period of time.

6. The breathing apparatus according to claim 5, **characterised in** that the error message (26) on the screen is devised so indicated programming can be verified when the area (30) assigned for this purpose on the screen (10) is touched.

7. The breathing apparatus (2), comprising a user interface (8) for programming the breathing apparatus's operating mode (10), said user interface (8) comprising an interactive screen (10) with a touch-sensitive surface capable of displaying at least active programming and indicated programming, devised so at least some programming can be performed when specially arranged segments on the screen (10) are touched one or more times, and a verification switch (12) for implementing active storage in the breathing apparatus (2) of the indicated programming displayed on the screen (10), **characterised in** that the user interface (8) is devised so no change in screen function is possible until the verification switch (12) has been actuated or indicated programming is identical to active programming.

8. Breathing apparatus according to claim 7, **characterised in** that the user interface (8) is devised so an error message (26) is displayed on the screen (10) if an attempt is made to change screen function without actuation of the verification switch (12) to implement active storage in the breathing apparatus (2) of indicated programming displayed on the screen.

9. The breathing apparatus according to claim 8, **characterised in** that the error message (26) on the screen (10) is devised so verification of indicated programming can be carried out by touching a surface (30) for this purpose on the screen (10).

10. The breathing apparatus (2) comprising a user interface (8) for programming the breathing apparatus's (2) operating mode, said user interface (8) comprising an interactive screen (10) with a touch-sensitive surface capable of displaying at least active programming and indicated programming, devised so at least some programming can be carried out when specially arranged segments on the screen (10) are touched one or more times, and a verification switch (12) for implementing active storage in the breathing apparatus (2) of the indicated programming displayed on the screen (10), **characterised in** that the user interface is devised so a message (32) comprising a request for verification by the operator is displayed on the screen (10) from the start of indicated programming until the verification switch (12) is actuated to implement active storage of indicated programming displayed on the screen (10).
